# EUROPEAN PATENT SPECIFICATION

(11) **EP 2 936 153 B1**
(45) Date of publication and mention of the grant of the patent: **28.11.2018**
(21) Application number: 13865805.9
(22) Date of filing: 17.12.2013
(51) Int. Cl.: G01N 33/74, C07K 16/00

(54) **SENSITIVE MULTIPLEX IMMUNOASSAY FOR SOLUBLE FIBROBLAST GROWTH FACTOR RECEPTORS**
SENSITIVE MULTIPLEX-IMMUNOASSAYS FÜR LÖSLICHE FIBROBLASTENWACHSTUMSFAKTORREZEPTOREN
TEST IMMUNOLOGIQUE MULTIPLEXE SENSIBLE POUR RÉCEPTEURS DE FACTEUR DE CROISSANCE DES FIBROBLASTES SOLUBLES

(30) Priority: 21.12.2012 US 201261740466 P
(43) Date of publication of application: 28.10.2015
(73) Proprietor: Janssen Biotech, Inc., Horsham, PA 19044 (US)
(72) Inventor: CHATURVEDI, Shalini, Spring House, Pennsylvania 19477 (US); PLATERO, Suso, Spring House, Pennsylvania 19477 (US); SIEGEL, Derick, Spring House Pennsylvania 19477 (US)
(74) Representative: Carpmaels & Ransford LLP
(86) International application number: PCT/US2013/075699
(87) International publication number: WO 2014/099933

(56) References cited:
- US-A1- 2003 008 410
- US-A1- 2005 239 108
- US-A1- 2007 248 605
- US-A1- 2009 258 369
- HANNEKEN A: "Structural characterization of the circulating soluble FGF receptors reveals multiple isoforms generated by secretion and ectodomain shedding", FEBS LETTERS, ELSEVIER, AMSTERDAM, NL, vol. 489, no. 2-3, 2 February 2001 (2001-02-02), pages 176-181, XP004248881, ISSN: 0014-5793, DOI: 10.1016/S0014-5793(00)02409-1
- HSIN-YUN HSU ET AL: "Suspension microarrays for the identification of the response patterns in hyperinflammatory diseases", MEDICAL ENGINEERING & PHYSICS., vol. 30, no. 8, 1 October 2008 (2008-10-01), pages 976-983, XP055265093, GB ISSN: 1350-4533, DOI: 10.1016/j.medengphy.2008.01.003 & HSIN-YUN HSU ET AL: "Multiplex microsphere-based flow cytometric platforms for protein analysis and their application in clinical proteomics â?? from assays to results", ELECTROPHORESIS, vol. 30, no. 23, 1 December 2009 (2009-12-01), pages 4008-4019, XP055265089, DE ISSN: 0173-0835, DOI: 10.1002/elps.200900211

## Description

### Field of the Invention

The subject matter described and claimed herein is in the field of analyte detection and specifically, relates to kits and methods to measure and identify soluble or extracellular domains of fibroblast growth factor receptors including receptor types 2, 3, and 4 (sFGFR2, sFGFR3 and sFGFR4) in biological samples.

### Background of the Invention

Carcinogenesis is a multistep process during which normal cells are transformed into cancer cells by accumulating several genetic mutations and acquiring several common features that promote the malignant phenotype, often referred to as the hallmarks of cancer. The six classic hallmarks of cancer include self-sufficiency in growth signals, insensitivity to antigrowth signals, limitless replication, evasion of apoptosis, sustained angiogenesis, and the ability to invade tissue and form metastasis (Hanahan, D., Weinber, R.A. Cell 2000, 100 : 57-70). Many of these features are caused by genetic alterations that involve the gain-of-function, amplification, and/or overexpression of key oncogenes together with the loss-of-function, deletion, and/or epigenetic silencing of tumor suppressors (Luo, J., Solimini, N.L., Elledge, S.J. Cell 2009, 136 :823-837). For example, several alterations and mutations have been identified within the family of fibroblast growth factor receptors (FGFRs) in a variety of human cancers. These mutations cause deregulation of FGFR signaling or overexpression of the receptors in cancers including breast, bladder, gastric, prostate, colon, multiple myeloma, and particularly lung cancer (Turner, N., Grose, R., Nat Rev Cancer. 2010, 10:116-129; Volm, M., Koomägi, R., Mattern, J., Stammler, G. Eur J Cancer. 1997, 33:691-693; Bergsagel, P.L., Kuehl, W.M. J Clin Oncol. 2005, 23: 6333-6338. Trudel, S., Stewart, A.K., Rom, E., Wei, E., Li, Z.H., Kotzer, S., Chumakov, I., Singer, Y., Chang, H., Liang, S.B., Yayon, A., Blood. 2006,107: 4039-4046).

Lung cancer is the leading cause of cancer-related death for both men and women, and accounted for 157,300 deaths in 2010 in the United States (Jemal, A., Siegel, J. Xu, J., Ward, E. CA Cancer J Clin. 2010, 60 : 277-300). The majority of lung cancer cases (approximately 85%) are classified as non-small cell lung cancer (NSCLC) for which survival has minimally improved in the last several decades (American Cancer Society. Cancer Facts & Figures, 2010, Atlanta, GA). Recently, novel treatments that target the epidermal growth factor receptor (EGFR) and vascular endothelial growth factor (VEGF) have emerged from an improved understanding of growth factor signaling in NSCLC. Although EGFR inhibitors exhibit some benefit in unselected patients, activating mutations in the EGFR identify a subset of tumors that are exquisitely sensitive to EGFR tyrosine kinase inhibitors (Lynch, T.J., Bell, D.W., Sordella, R., Gurubhagavatula, S., Okimoto, R.A., Brannigan, B.W., Harris, P.L., Haserlat, S.M., Supko,J.G., Haluska, F.G., Louis, D.N., Christiani, D.C., Settleman, J., Haber, D.A.N Engl J Med. 2004, 350: 2129-2139; Shepherd, F.A., Pereira, J.R., Ciuleanu, T., Eng, H.T., Hirsh, V., Thongprasert, S., Campos, D., Maoleekoonpiroj, S., Smylie, M., Martins, R., Van Kooten, M., Dediu, M., Findlay, B., Tu, D., Johnston, D., Bezjak, A., Clark, G., Santabárbara, P., Seymour, L. N Engl J Med. 2005, 353 : 123-132.). Similarly, anti-VEGF treatment produces an improved outcome for certain patients with NSCLC (Sandler, A. , Gray, R., Perry, M.C., Brahmer, J., Schiller, J.H., Dowlati, A., Lilenbaum, R., Johnson, D.H. N Engl J Med. 2006, 355 : 2542-2550.), although defining predictive biomarkers for anti-VEGF therapeutic efficacy has been less successful (Ramalingam, S.S., Belani, C.P. Curr Opin Oncol. 2010, 22 : 79-85). The experience with EGFR and VEGF inhibitors in NSCLC highlights the need to define other signaling pathways involved in tumor progression, angiogenesis, and resistance to currently available treatments (Siegel, R., Naishadham, D., Jemal, A. CA Cancer J Clin. 2012, 62: 10-29).

Fibroblast growth factor receptors (FGFRs) are transmembrane tyrosine kinase receptors that are involved in multiple physiologic processes including angiogenesis, organogenesis, tissue development, and endocrine signaling. Structurally, FGFRs consist of a signal peptide that is cleaved off, three immunoglobulin (Ig)-like domains, an acidic box, a transmembrane domain, and a split tyrosine kinase domain (Turner, N., Grose, R. Nat Rev Cancer. 2010, 10 : 116-29; Beenken, A., Mohammadi, M. Nat Rev Drug Discov. 2009, 8: 235-253). Thus far, four genes have been identified to code for FGFRs named FGFR1, FGFR2, FGFR3, and FGFR4. Each type of FGFR varies in sensitivity and specificity. The system is further complicated by extensive splicing that can occur in FGFR1-3 mRNAs, generating a multitude of variants with different ligand-binding and signal-transducing activities (Turner, N., Grose, R. Nat Rev Cancer. 2010, 10 :116-29; Beenken, A., Mohammadi, M. Nat Rev Drug Discov. 2009, 8: 235-253). The fibroblast growth factors (FGFs) are the ligands for FGFRs and consist of twenty two soluble excreted peptides, which are released depending on cellular needs. Together, FGFs and FGFRs create an extremely high number of ligand/receptor combinations enabling precise fine-tuning of growth signals. The regulation of FGF signaling involves several mechanisms including the release of soluble forms of FGFRs (sFGFRs), generated by proteolytic cleavage at the cell surface by metalloproteases (Müllberg J, Althoff K, Jostock T, Rose-John S. Eur Cytokine Netw. 2000, 11: 27-38). The shedding of these receptors provides a key mechanism for downregulation of FGF signaling and the release of biologically active proteins into circulation (Peschon J.J., Slack J.L., Reddy P., Stocking K.L., Sunnarborg, S.W., Lee, D.C., Russell, W.E., Castner, B.J., Johnson, R.S., Fitzner, J.N., Boyce, R.W., Nelson, N., Kozlosky, C.J., Wolfson, M.F., Rauch, C.T., Cerretti, D.P., Paxton, R.J., March, C.J., Black, R.A. Science. 1998, 282: 1281-2184).

There is mounting evidence in the last twenty years that shows the existence of soluble FGFR receptors in the blood at particularly high levels in cancer patients (Hanneken, A., Ying, W., Ling, N., Baird, A. Proc Natl Acad Sci. 1994, 91: 9170-9174; Hanneken A. FEBS Lett. 2001, 489: 176-181), suggesting that monitoring the presence of these receptors in circulation may be important to understand the progression and/or treatment of cancer. For example, a C-terminally truncated soluble form of FGFR4 is expressed by human epithelial breast cancer cell lines that can functionally modulate FGF action (Ezzat, S,, Zheng, L., Yu, S., Asa, S.L. Biochem Biophys Res Commun. 2001,287: 60-65). In addition, an alternatively spliced form of FGFR3 has been identified in a human squamous carcinoma cell line, which has the capacity to bind to FGF1 and FGF2, as shown by cross-linking experiments (Terada, M., Shimizu, A., Sato. N., Miyakaze, S.I., Katayama, H., Kurokawa-Seo. M. Mol Cell Biol Res Commun. 2001, 4: 365-373). The ligand-binding affinities of these secreted sFGFRs are often similar to the membrane-bound form of these receptors, suggesting that their biological role is to modulate the action of ligands by competing with the cell surface receptor.

Although the FGFRs in some of these studies have been identified in the blood or cell medium, the only method that has been utilized in the prior art is immunoblotting (Hanneken, A., Ying, W., Ling, N., Baird, A. Proc Natl Acad Sci. 1994, 91: 9170-9174 ; Hanneken A.FEBS Lett. 2001, 489: 176-181), a technique that is not sensitive, linear, or diagnostic. Accordingly, there is an unmet need for the development of a standardized, fast, precise, and economical screen for sFGFR, which is amenable to automation. Since commercially available DuoSet® antibodies are available to detect soluble forms of total FGFR2, FGFR3, and FGFR4 the applicants used the ELISA method to develop a detection assay for sFGFRs similar to that used to detect soluble epidermal growth factor receptors (Muller, V., Witzel, I., Pantel, K., Krenkel, S., Luck, H.J., Neumann, R., Keller, T., Dittmer, J., Janicke, F., Thomssen, C. Anticancer Res. 2006, 26 : 1479-1487). However, the DuoSet ELISA system was unsatisfactory because only the soluble FGFR3 (sFGFR3) was detected in the assayed biological samples. The applicants then transferred the assay to a Meso Scale Discovery format to increase the detection sensitivity for soluble FGFR2 (sFGFR2) and FGFR4 (sFGFR4). However, sFGFR2 and sFGFR4 remained undetected. Therefore, a completely different assay system was successfully developed to detect SFGFR2, SFGFR3, and sFGFR4 in biological samples, which utilizes a multiplex, microsphere-based platform. The subject matter disclosed herein provides the kits and methods for detection of sFGFR2, sFGFR3, and sFGFR4 in biological samples.

### Summary of the Invention

The present invention is directed to a kit to detect soluble fibroblast growth factor receptors (sFGFRs) in a biological sample, comprising:
a capture antibody composition specifically binding sFGFRs conjugated to the microsphere; and
a detection antibody composition specifically binding sFGFRs conjugated to a detection label;
wherein 0.14 - 494 ng/mL sFGFR2, 0.023 - 74 ng/mL of FGFR3, and 0.033 - 123 ng/mL of FGFR4 is detected in the biological sample;
wherein the capture antibody composition comprises a capture antibody specifically binding FGFR2, a capture antibody specifically binding FGFR3, and a capture antibody specifically binding FGFR4; and
wherein the detection antibody composition comprises a detection antibody specifically binding FGFR2, a detection antibody specifically binding FGFR3, and a detection antibody specifically binding FGFR4.

The present invention is also directed to a method for measuring the amount of sFGFR2, sFGFR3 and sFGFR4 in a biological sample, comprising:
a. contacting the biological sample with a capture antibody composition specifically binding sFGFR2, sFGFR3, and sFGFR4 conjugated on a microsphere;
b. contacting the biological sample with a detection antibody composition specifically binding sFGFR2, sFGFR3, and sFGFR4 coupled to a detection label;
c. measuring the signal produced by the detection label;
d. correlating the amount of the signal produced in c. to the amount of sFGFR2, sFGFR3 or sFGFR4 in the biological sample.

Cancer patients typically have higher soluble FGF receptor content in their biological fluids and tissues as compared to non-diseased patients. The kits and methods described herein enable those skilled in the art to identify and quantify multiple isoforms of soluble FGF receptors in such patients and provide a basis for identifying and quantifying the FGF content in oncology patients. Another embodiment of the inventions described herein also pertains to a multiplexing microsphere technology for simultaneous screening of sFGFRs.

The kit of the present invention is used to detect sFGFR2, sFGFR3, and sFGFR4. The kit comprises a capture antibody composition, comprising a capture antibody specifically binding sFGFR2, a capture antibody specifically binding sFGFR3, and a capture antibody specifically binding sFGFR4 that is conjugated to a microsphere. The microsphere having the conjugated capture antibody has a diameter of about 5 µm and two fluorophores. Each fluorophore being spectrally distinct from the other fluorophore. The kit further comprises a detection antibody composition, comprising a detection antibody specifically binding sFGFR2, a detection antibody specifically binding sFGFR3, and a detection antibody specifically binding sFGFR4 that is conjugated to any detectable label known in the art suitable for the described intended purpose. Furthermore, the kit may comprise a blocking buffer and an incubation buffer.

The kit of the present invention detects predefined threshold levels of sFGFR2, sFGFR3 and/or sFGFR4, wherein the predefined threshold levels are 0.14 - 494 ng/mL sFGFR2, 0.023 - 74 ng/mL of sFGFR3, and 0.033 - 123 ng/mL of sFGFR4 is detected in the biological sample. Preferably, such levels are detected at levels having values as follows: at least 0.14-494 ng/mL of sFGFR2, 0.23-74 ng/mL of sFGFR3, and 0.33-123 ng/mL of sFGFR4 in a biological sample. The kit comprises capture antibodies for sFGFR2, sFGFR3, and sFGFR4 conjugated to microspheres and detection antibodies for sFGFR2, sFGFR3 and sFGFR4 conjugated to a detection label known in the art. The kit may further comprise a blocking buffer and an incubation buffer.

The present invention is also directed to a method for measuring the amount of sFGFR2, sFGFR3, and sFGFR4 in a biological sample. The method comprises contacting the biological sample with a capture antibody composition specifically binding sFGFR2, sFGFR3 and sFGFR4 conjugated on a microsphere, contacting the biological sample with a detection antibody composition specifically binding sFGFR2, sFGFR3 and sFGFR4 coupled to a detection label known in the art, measuring the signal produced by the detection label and correlating the amount of signal produced to the amount of sFGFR2, sFGFR3 and sFGFR4 in the biological sample.

### Abbreviations

- FGF: fibroblast growth factor
- FGFR: fibroblast growth factor receptor
- FGFRs: fibroblast growth factor receptors
- sFGFR: soluble fibroblast growth factor receptor
- sFGFR 2: soluble fibroblast growth factor receptor 2
- sFGFR3: soluble fibroblast growth factor receptor 3
- sFGFR4: soluble fibroblast growth factor receptor 4
- sFGFRs: soluble fibroblast growth factor receptors
- NSCLC: Non-small cell lung carcinoma
- ADAMs: adamalysin
- ELISA: enzyme-linked immunosorbant assay
- MSD: Meso Scale Discovery
- EDTA: ethylenediaminetetraacetic acid
- BSA: bovine serum albumin
- LDD: least detectable dose
- CV: coefficient of variation
- LLOQ: lower limit of quantification
- RTKI: receptor tyrosine kinase inhibitors
- VEGFs: vascular endothelial growth factors
- PDGFRs: platelet-derived growth factor receptors

### Definitions

The term "kit" as used herein refers to a combination of reagents and other materials. It is contemplated that the kit may include reagents such as buffering agents, protein stabilizing reagents, signal producing systems (*e.g*., florescence signal generating systems), antibodies, control proteins , as well as testing containers (*e.g*., microtiter plates, etc.). It is not intended that the term "kit" be limited to a particular combination of reagents and/or other materials. In one embodiment, the kit further comprises instructions for using the reagents. The test kit may be packaged in any suitable manner, typically with the elements in a single container or various containers as necessary along with a sheet of instructions for carrying out the test. In some embodiments, the kits also preferably include a positive control sample. Kits may be produced in a variety of ways known in the art.

The term "biological sample" encompasses a variety of sample types obtained from an organism and can be used in a diagnostic or monitoring assay. The term encompasses blood and other liquid samples of biological origin, solid tissue samples, such as a biopsy specimen or tissue cultures or cells derived therefrom and the progeny thereof. The term encompasses samples that have been manipulated in any way after their procurement, such as by treatment with reagents, solubilization, or enrichment for certain components. The term encompasses a clinical sample, and also includes cells in cell culture, cell supernatants, cell lysates, serum, plasma, biological fluids, and tissue samples. The term "biological sample" is meant to distinguish between a sample in a clinical setting from a sample that may be a recombinant sample or derived from a recombinant sample.

The terms "capture antibody" and "detecting antibody" in the sense of the present application are intended to encompass those antibodies which bind to a specific sFGFR with a high affinity.

The term "antibody" is used in the broadest sense and specifically covers intact monoclonal antibodies, polyclonal antibodies, multispecific antibodies (e.g. bispecific antibodies) formed from at least two intact antibodies, and antibody fragments as long as they exhibit the desired biological poperty. The antibody may be derived from any species and may be IgM, IgG (e.g. IgG1, IgG2, IgG3, or IgG4), IgD, IgA, or IgE, for example. The desired biological activity is binding to sFGFR.

The term "monoclonal antibody" as used herein refers to a preparation of antibodies of single molecular composition. A monoclonal antibody composition displays a single binding specificity and affinity for a particular epitope of sFGFRs.

The term "polyclonal antibody" as used herein refers to a composition of different antibody molecules which is capable of binding to or reacting with several different specific antigenic determinants on the same or on different antigens. The variability in antigen specificity of a polyclonal antibody is located in the variable regions of the individual antibodies constituting the polyclonal antibody, in particular in the complementarity determining regions (CDR)1, CDR2 and CDR3 regions. Preferably, the polyclonal antibody is prepared by immunization of an animal with the target sFGFR or portions thereof. Alternatively, the polyclonal antibody may be prepared by mixing multiple monoclonal antibodies having desired specificity to a target sFGFR.

The term "specifically binding" refers to binding between two molecules, for example, an antigen and an antibody, characterized by the ability of a molecule (antigen) to associate with another specific molecule (antibody) even in the presence of many other diverse molecules, i.e., to show preferential binding of one molecule for another in a heterogeneous mixture of molecules.

The term "microsphere" refers to a small discrete solid support. These discrete solid supports can be used for attachment of a population of probes such that each individual probe in the population differs from each other. The composition of a microsphere can vary, depending on, for example, the format, chemistry and/or method of attachment and/or on the method of nucleic acid synthesis. Exemplary microsphere compositions include solid supports, and chemical functionalities imparted thereto, used in polynucleotide, polypeptide and/or organic moiety synthesis. Such compositions include, for example, plastics, ceramics, glass, polystyrene, methylstyrene, acrylic polymers, paramagnetic materials, thoria sol, carbon graphite, titanium dioxide, latex or cross-linked dextrans such as sepharose, cellulose, nylon, cross-linked micelles and Teflon™, as well as any other materials.

The term "fluorophore" as used herein refers to any fluorescent compound or protein that can be used in the quantification and detection of the sFGFR antigens.

The term "sensitivity" essentially is a measure of how well the multiplex assay described in the present invention correctly identifies a specific sFGFR at its minimum concentration. As described herein, sensitivity is defined as the least detectable dose (ng/mL) of a specific sFGFR which can be accurately recovered between 80-120%.

The term "buffer" refers to either a buffered solution or to water. The buffer supports a reaction between the analyte of interest (*e.g*., sFGFR) and the analyte binding agent (*e.g*., sFGFR antibody) and does not interfere with antibody/antigen interaction.

The term "living subject" as used herein refers to any living organism, including human.

The terms "a" or "an" as used herein in the specification may mean one or more. As used herein in the claim(s), when used in conjunction with the word "comprising", the words "a" or "an" may mean one or more than one. As used herein "another" may mean at least a second or more.

The term "comprise," or variations such as "comprises" or "comprising," as used herein may be used to imply the inclusion of a stated element or integer or group of elements or integers, but not the exclusion of any other element or integer or group of elements or integers.

Unless defined otherwise, all technical and scientific terms used herein have the same meaning as commonly understood by one of ordinary skill in the art to which this invention is related.

### Brief Description of Figures

Figure 1: Validation parameters established for serum and plasma using the assay developed for soluble FGFR2, FGFR3, and FGFR4.
Figure 2: Spike-recovery for FGFR2 at concentrations 7.8, 2.9, 1.7, and 0.82 ng/mL in serum and plasma (N=3).
Figure 3: Spike-recovery for FGFR3 at concentrations 1.4, 0.57, 0.30, and 0.15 ng/mL in serum and plasma (N=3).
Figure 4: Spike-recovery for FGFR4 at concentrations 2.2, 0.94, 0.54, and 0.29 ng/mL in serum and plasma (N=3).
Figure 5: Cross-Reactivity of: FGFR3 and FGFR4 with anti-FGFR2 as capture, FGFR2 and FGFR4 with anti-FGFR3 as capture, and FGFR2 and FGFR3 with anti-FGFR4 as capture
Figure 6: Standard Curves (N=5) for FGFR2 with nominal concentration in ng/mL versus back calculated concentrations in ng/mL.
Figure 7: Standard Curves (N=5) for FGFR3 with nominal concentration in ng/mL versus back calculated concentrations in ng/mL.
Figure 8: Standard Curves (N=5) for FGFR4 with nominal concentration in ng/mL versus back calculated concentrations in ng/mL.
Figure 9: Experimental assessment of serum concentration of FGFR2 in control samples across runs spiked with low, medium, and high concentrations of the analytes (N=5).
Figure 10: Experimental assessment of serum concentration of FGFR3 in control samples across runs spiked with low, medium, and high concentrations of the analytes (N=5).
Figure 11: Experimental assessment of serum concentration of FGFR4 in control samples across runs spiked with low, medium, and high concentrations of the analytes (N=5).
Figure 12: Spike-recovery data in serum (N=3) and plasma (N=3) at concentrations 7.8, 2.9, 1.7, and 0.82 ng/mL for FGFR2, concentrations 1.4, 0.57, 0.30, and 0.15 ng/mL for FGFR3 and concentrations 2.2, 0.94, 0.54, and 0.29 ng/mL for FGF R4.
Figure 13: Dilutional linearity data for FGFR2 in plasma, serum and a high-spiked control
Figure 14: Dilutional linearity data for FGFR3 in plasma, serum and a high-spiked control
Figure 15: Dilutional linearity data for FGFR4 in plasma, serum and a high-spiked control
Figure 16: Top Panel: Sample buffers used to establish a better range in prostate cancer serum and lung cancer serum. Bottom Panel: Different blocking buffers tried in order to get a higher signal for metastatic cancer serum.
Figure 17: Reference range for FGFR2, FGFR3, and FGFR4 in prostate cancer (N=5); and lung cancer (N=10).

### Detailed Description of the Invention

Early analysis of soluble FGFR concentrations is beneficial to understand the status of a cancer (before and after treatment) and would help in the selection of specific drug candidates targeting FGFRs. Such an analysis would also render clues about the mode of action of a FGFR drug. Therefore, it is imperative to have a suitable diagnostic assay, which is sensitive and specific for FGFRs.

Previous attempts to obtain a suitable assay that reliably detects multiple isoforms of sFGFR failed despite proving successful in the detection of other soluble growth factor receptors. Surprisingly, the use of a microsphere-based multiplex immunoassay technology (specifically the "Luminex 100/200™" platform described herein and in U.S. Patent Nos. 6,599,331, 6,592,822, and 6,268,222, enabled development of an assay to accurately measure sFGFR2, sFGFR3, and sFGFR4 in biological samples. The microsphere-based multiplex immunoassay technology is a sandwich assay. The term "sandwich assay" refers to an immunoassay where the antigen is sandwiched between two binding reagents, which are typically antibodies. The first binding reagent (capture antibody) being attached to a microsphere and the second binding reagent (detection antibody) comprising a detectable group. Examples of detectable groups include, for example and without limitation: fluorochromes, enzymes, epitopes for binding a second binding reagent (for examples, when the second binding reagent/antibody is a mouse antibody, which is detected by a fluorescently labeled anti-mouse antibody), for example an antigen or a member of a binding pair, such as biotin.

The polysterene microspheres are important components of this assay. These microsphere beads are dyed internally with two spectrally distinct fluorophores. Using precise ratios of these fluorophores, an arrays is created consisting of 100 different microsphere sets with specific spectral addresses. Each microsphere set can possess a different reactant on its surface. In this application, the reactant is referred to as "capture antibody". Because microsphere sets can be distinguished by their spectral addresses, they can be combined, allowing up to 100 different analytes to be measured simultaneously in a single reaction vessel. A third fluorophore coupled to a reporter molecule- in this application referred to as "detection antibody" - quantifies the biomolecular interaction that has occurred at the microsphere surface. Microspheres are interrogated individually in a rapidly flowing fluid stream as they pass by two separate lasers in the Luminex analyzer. High speed digital signal processing classifies the microsphere based on its spectral address and quantifies the reaction on the surface in a few seconds per sample reaction.

For the assays described herein, the microsphere-based multiplex immunoassay offer several advantages :
- The costs of the assay are inexpensive because a separate kit is not required for each individual sFGFR.
- Since one kit can test multiple sFGFRs, the amount of biological sample required to run an assay is greatly reduced.
- The quantitation measurements of the assay offer high reproducibility because pre-processing or titering of the biological sample is not required.
For these reasons, although other immunoassays, such as radioimmunoassay are capable of use in the context of the present invention, the microsphere-based multiplex immunoassay is preferred.

### EXAMPLES

The following non-limiting examples are provided to further illustrate the present invention. It will be appreciated by those of skill in the art that the techniques disclosed in the examples that follow represent approaches the inventors have found function well in the practice of the claimed inventions, and thus should be considered to constitute examples of modes for its practice. However, those skilled in the art should, in light of the present disclosure, appreciate that many changes can be made in the specific embodiments that are disclosed and still obtain a like or similar result without departing from the scope of the invention.

### EXAMPLE 1: Collection and Storage of Biological Samples

Since sFGFRs are mostly released into circulation after proteolytic cleavage, blood, serum and plasma samples [Bioreclamation, LLC (Westbury, NY)] were used to develop and validate the multiplex immunoassay. Blood samples were collected from the antecubital area of consenting healthy volunteers by state-licensed phlebotomists and allowed to clot in 450-500 mL dry collection bags in a refrigerator overnight. Sera were separated by centrifugation at 2800 x g for twenty minutes at 5 °C. The separated sera were transferred into transfer bags using a plasma extractor device to ensure no red blood cell contamination. Human plasma was collected in collection bags containing potassium-EDTA, mixed gently for five minutes and allowed to sit at room temperature for thirty minutes. The bags were centrifuged at 2800 x g for twenty minutes at 5 °C. The separated plasma was transferred into transfer bags using a plasma extractor device to ensure no red blood cell contamination. Both the human sera and human plasma were stored at 4 °C for up to one week before being separated into aliquots in cryo tubes and frozen at -80 °C until use.

### EXAMPLE 2: Development of the Multiplex Immunoassay

Successful implementation of a microsphere-based multiplex immunoassay for sFGFR detection in biological samples requires protocol development and optimization. First, the reagents that will result in the most sensitive assay must be identified. The first set of reagents that was evaluated was the capture and detection antibody pairs for sFGFR2, sFGFR3 and sFGFR4. Several different sources of antibodies were explored; however, the antibody pairs listed in Table 1 rendered the best range of detection for sFGFR2, sFGFR3, and sFGFR4 and were therefore used in the multiplex system.

**Table 1: Combination of capture and detection antibodies used to set up the assay for FGFR2, FGFR3, and FGFR4.**

| | Antibody Summary | | |
|---|---|---|---|
| | FGF R2 | FGF R3 | FGF R4 |
| Capture: | Abcam (Cambridge, MA) ab89476 | R&D Systems (Minneapolis, MN) MAB7661 | R&D Systems Duoset 842983 |
| Detection (total): | R&D Systems MAB6841 | R&D Systems MAB766 | R&D Systems Duoset 842741 |
| Standards: | All standards were obtained from corresponding R&D Systems Duoset kits | | |

The next component of the assay that was prepared was the capture antibody-coupled microspheres. Capture antibodies were carboxylated («-COOH ») specifically to polystyrene 5.6 micron microspheres as follows: anti-FGFR2 to bead #(88), anti-FGFR3 to bead #(16), and anti-FGFR4 to bead #(79). These regions correspond to 3 of the 100 uniquely dyed potential regions used by the Luminex 100/200™ system. The coupling of the target capture antibodies to the polystyrene microspheres was performed using a proprietary conjugation system, which uses a procedure similar to the Bio-Plex® Amine Coupling kit from Bio-Rad Life Sciences (Cat # 171-406001). Briefly, the covalent couple of the target capture antibody to the carboxylated polystyrene microsphere is achieved via carbodiimide reactions involving the protein primary amino groups and the carboxyl functional groups bound on the surface of polystyrene microspheres. The covalent attachment is permanent, leaving no unbound protein after cleanup, even after long periods of storage. The detection antibodies for sFGFR2 and sFGFR3 were biotinylated using Sulfo-NHS-LC-Biotin (Pierce, Cat #21327) according to the manufacturer's protocol, while the detection antibody for sFGFR4 was purchased with the biotin label already attached.

Another set of reagents evaluated was the buffers to be used for sample dilutions, blocking steps and incubations. Typical buffers utilized in multiplex assays are PBS-based or Tris-based. The buffer that worked best for incubations and dilutions was PBS with 1% BSA and for nonspecific protein blocking was PBS with 1% Bovine Gamma Globulin and Immunoglobulin G.

Once the reagents for the immunoassay were prepared, the following protocol was used to quantitate sFGFR2, sFGFR3 and sFGFR4 in biological samples:
- Five µL of diluted capture antibody-labeled microspheres for all three sFGFRs (1800 beads per analyte per well) mixed with five µL of a blocker consisting of 1% Bovine Gamma Globulin (MP Biomedicals, Santa Ana, CA, Catalog # 82041) in PBS with added Immunglobulin G.
- This mixture was added to a 96 well flat bottom microtiter plate.
- The sample to be used was thawed at room temperature immediately before testing.
- Ten µL of standard, quality control, or serum diluted 1:5 in a 4% BSA buffer containing a preservative (sodium azide) were added to the plate, samples were mixed with a pipette and the plate was incubated at room temperature for one hour without shaking.
- Ten µL of biotinylated detection antibodies (diluted in a 1% BSA buffer with preservative to a final concentration of 3 µg/mL for all three analytes) were added to each well, thoroughly mixed, and incubated for one hour at room temperature.
- Ten µL of streptavidin-phycoerythrin diluted to 100 µg/mL was added to each well, thoroughly mixed, and incubated for thirty minutes at room temperature.
- A filter-membrane microtiter plate was pre-wetted with 100 µL of a BSA-based wash buffer with sodium azide followed by aspiration via a vacuum manifold device.
- The reaction contents in the flat-bottomed microtiter plate were transferred to the respective wells of the filter plate. All the wells were vacuum-aspirated and the contents are washed twice with 100 µL of wash buffer.
- After the final wash, 100 µL of wash buffer was added to each well and the microspheres were resuspended by thorough mixing.
- The plate is then analyzed on a Luminex 100/200™ reader which uses a proprietary analysis software. For each plate, a standard curve is created for each sFGFR using proprietary software. The standard curves for sFGFR2 and sFGFR3 use a 5-Parameter Logistic regression and the standard curve for sFGFR4 uses a 4-Parameter Logistic regression.

### EXAMPLE 3: Statistical Validation of the Multiplex Immunoassay

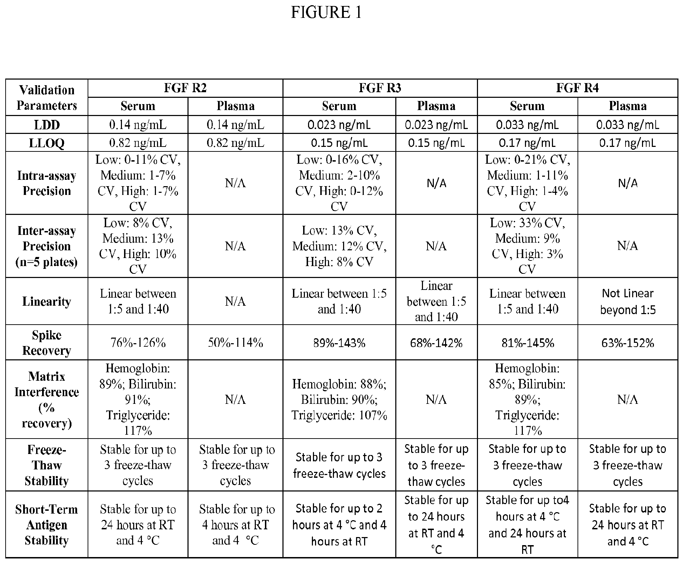

On the basis of guidelines from the US Food and Drug Administration (U.S. Department of Health and Human Services, Center for Drug Evaluation and Research (CDER), and Center for Veterinary Medicine (CVM). Guidance for Industry. Bioanalytical method validation. May 2001. http://www.fda.gov/downloads/Drugs/GuidanceComplianceRegulatoryInformation/ Guidances/UCM070107.pdf), a validation program was developed that entailed full validation of the sFGFR2, sFGFR3, and sFGFR4 multiplex immunoassay. The program included assessment of best sample matrix, assay selectivity, linear range as determined by a standard curve, least detectable dose (LDD), lower limit of quantification (LLOQ), precision, absolute recovery, dilutional linearity, matrix interference, freezing/thawing stability, stability at room temperature, and reference range (FIG. 1).

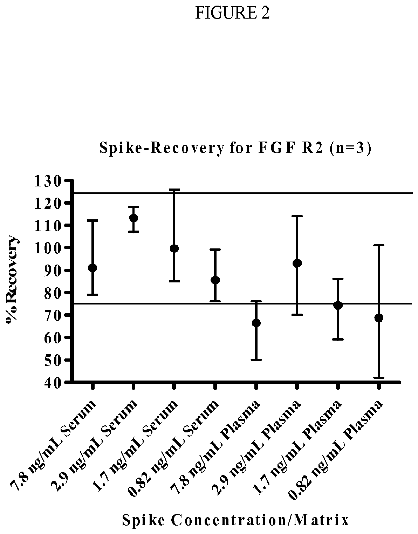

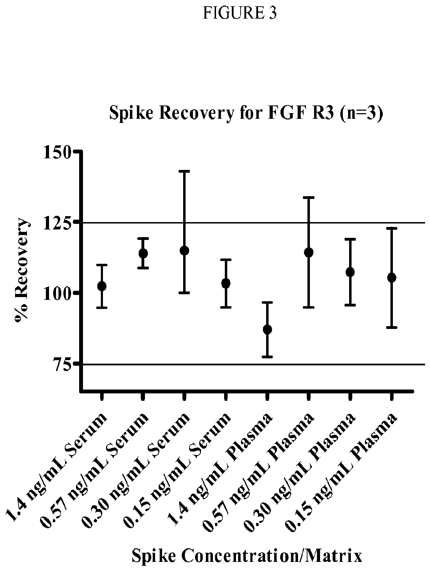

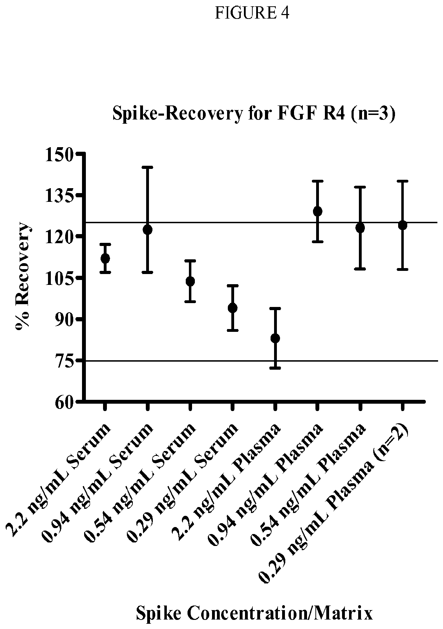

**Comparison of plasma and serum samples** : Since blood was being used to measure the content of sFGFR2, sFGFR3 and sFGFR4 in the assay, a comparison was made between plasma or serum to identify the best biological matrix. As seen in FIG. 2, FIG. 3 and FIG. 4, sFGFR recovery was consistently better when assaying serum. Therefore, serum was the final choice of matrix for further validation analyses. The multiplex immunoassay methods described in the present invention is also amenable to other biological samples (cerebrospinal fluid, urine, lymph, saliva, sudor, pleura fluid, synovial fluid, aqueous fluid, tear fluid, bile, pancreas secretion), although a fit-for-purpose validation will also need to be established to identify the best choice of matrix.

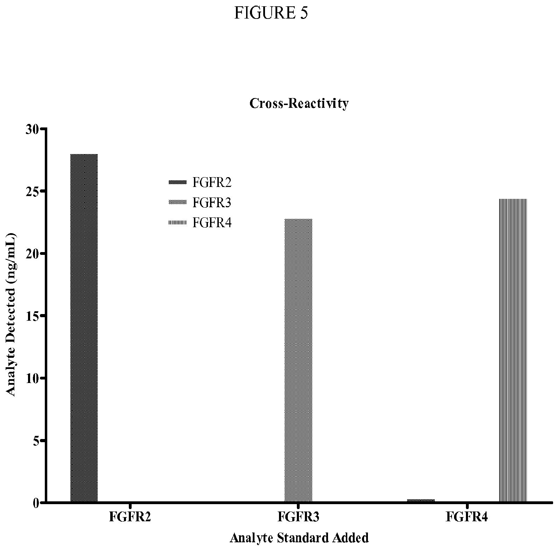

**Assay Selectivity:** Since sFGFR2, sFGFR3, and sFGFR4 were tested in the same sample well, it was important to ascertain that there is no significant cross-reactivity between the three analytes. In order to investigate cross-reactivity, the FGFR2 capture antibody was tested against the highest concentration of sFGFR3 and sFGFR4 for signal. Similarly, all other combinations were evaluated and only 1.6% cross-reactivity was found between the FGFR4 capture antibody and sFGFR2 at the highest concentration (FIG. 5).

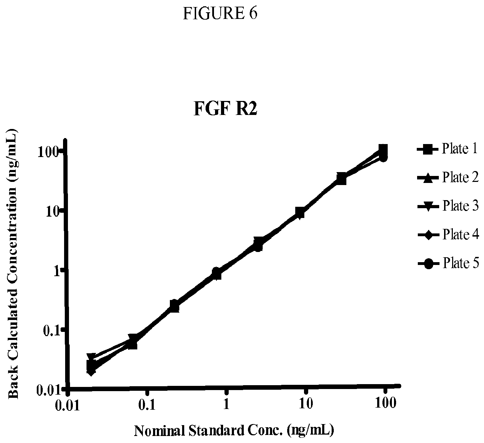

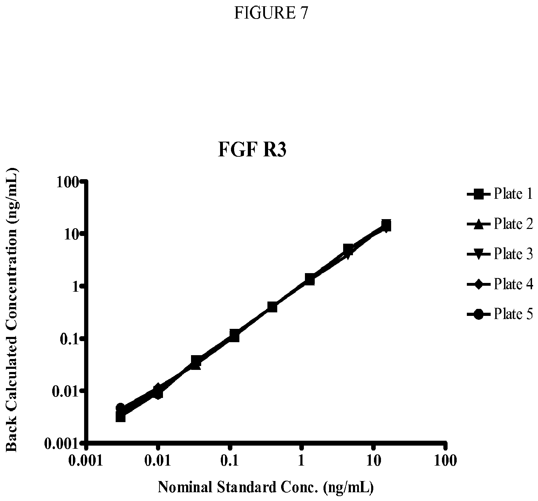

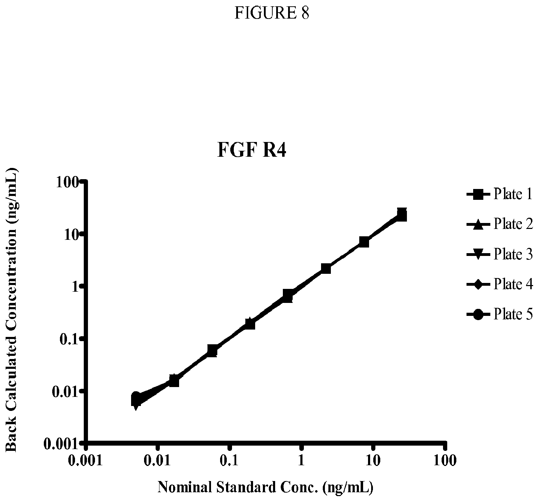

**Standard Curves**: Total FGFR2a standard (part #841650), total FGFR3 standard (part #841879) and total FGFR4 standard (part #842742) available from R&D Systems Duosets (Catalog numbers DYC665, DYC766 and DYC685, respectively) were used to generate the standard curves. Standard curves for each sFGFR were made by first creating a high standard mix of the three standards in a fish serum/BSA buffer: 100 ng/mL for FGFR2, 15 ng/mL for FGFR3 and 25 ng/mL for FGFR4. This high standard was serially diluted seven times by adding 37 µL of each standard with 88 µL of standard diluent. The standard curve ranges of FGFR2, FGFR3 and FGFR4 were 100 ng/mL - 0.020 ng/mL, 15 ng/mL - 0.003 ng/mL and 25 ng/mL - 0.005 ng/mL, respectively. Standard curves for each analyte were run on every plate, and the values were averaged, with percent CV calculated across runs (FIG. 6, FIG. 7 and FIG. 8). The average CV for sFGFR2, sFGFR3, and sFGFR4 were 8.38%, 6.38%, 5.13%, respectively, indicating that the curves had very minimal variation from plate to plate.

**Least Detectable Dose**: The lowest concentration of analyte that can be discriminated from the blank with 99% confidence is known as the least detectable dose (LDD). LDD was calculated by averaging the signal from 20 replicate determinations of the standard curve blank and adding three standard deviations to the average blank signal.

**Lower Limit of Quantification (LLOQ)**: The lower limit of quantitation (LLOQ) is the lowest standard that can be detected accurately. In order to fit within the LLOQ definition, the standard must be linear, fall within the range of the assay within a CV of 30%, and must recover between 75-125% when spiked in the matrix. LLOQ was determined by running two-fold dilutions of low standards in triplicate over three runs. Standards 5 - 8 were diluted two-fold and assayed in triplicates over three different runs. The CV was calculated and plotted against concentration. The LLOQ, interpolated from this plot and multiplied by the dilution factor, was calculated to be 0.53 ng/mL, 0.084 ng/mL, and 0.15 ng/mL, respectively for sFGFR2, sFGFR3, and sFGFR4. As mentioned, it is also important for the analyte to recover within 75 - 125% at the concentration of the LLOQ. However, samples could not be successfully recovered for sFGFR2 at concentrations below 0.82 ng/mL. The average recovery at 0.82 ng/mL for serum (N=3) was determined to be 85.67%. FGFR3 samples could not be successfully recovered at concentrations below 0.15 ng/mL, and the average recovery of serum samples (N=3) spiked with sFGFR3 was 103.3%. FGFR4 samples could not be successfully recovered at concentrations below 0.17 ng/mL, and the average recovery of serum samples (N=3) spiked with sFGFR3 was 101.3%. Finally, the LLOQ determined for sFGFR2, sFGFR3, and sFGFR4 was 0.82 ng/mL, 0.15 ng/mL, 0.17 ng/mL, respectively (Table 2).

**Table 2: LLOQ and dynamic range for FGFR2, FGFR3, and FGFR4.**

| **ANALYTE** | **Units** | **LLOQ** | **Dynamic Range** |
|---|---|---|---|
| **FGF-R2** | ng/mL | 0.82 | 0.10 - 494 |
| **FGF-R3** | ng/mL | 0.15 | 0.015 - 74 |
| **FGF-R4** | ng/mL | 0.17 | 0.025 - 123 |

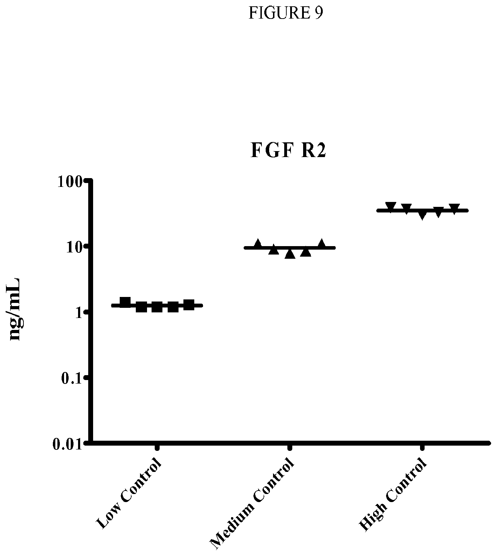

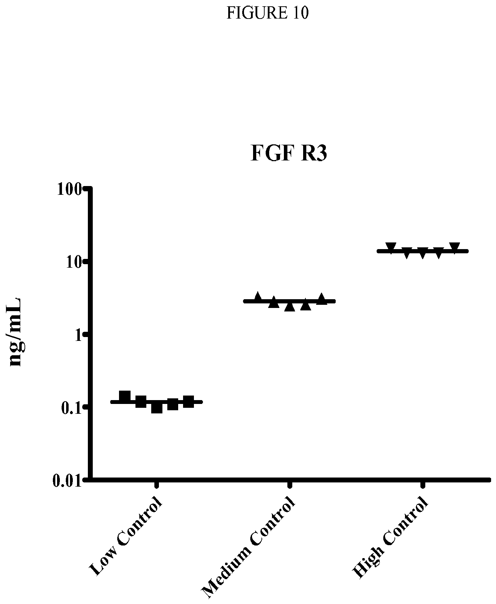

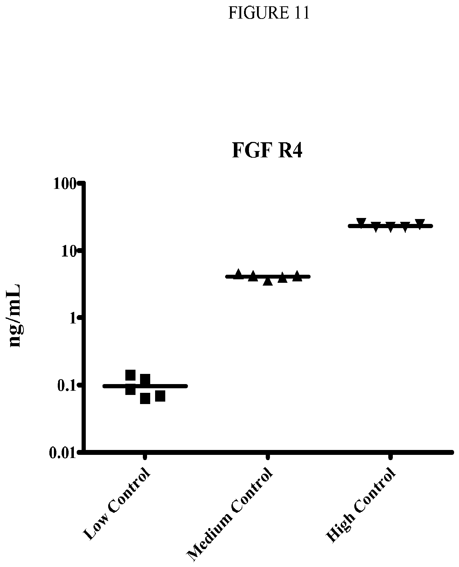

**Precision**: Precision for the assay was determined within a run (intra-assay CV) and over a series of runs (inter-assay CV) by measuring three levels of controls in duplicate over a minimum of five runs. The average values for sFGFR2 controls were 1.3, 9.3, and 35 ng/mL with inter assay CV ranging from 8 - 13%, and intra-assay CV ranging from 1 - 11% (FIG. 9). The average values for sFGFR3 controls were 0.117, 2.8, and 14 ng/mL with inter assay CV ranging from 8 - 13%, and intra-assay CV ranging from 0 - 16% (FIG. 10). The average values for sFGFR4 controls were 0.095, 4.1, and 23 ng/mL with inter assay CV ranging from 3 - 33%, and intra-assay CV ranging from 1 - 21% (FIG. 11). The upper range for sFGFR4 is a little higher than 30%, which is a result of one bad run having a high CV of 21%. The reason for a high CV is because the average concentration of that sample is 0.095 ng/mL, which is much lower than the LLOQ for sFGFR4.

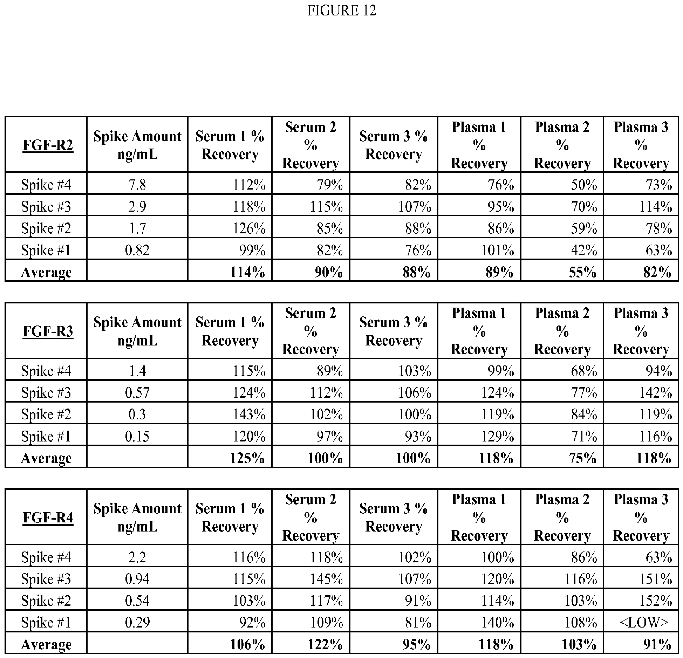

**Absolute Recovery:** In order to determine the percent recovery of each sFGFR, serum was spiked with sFGFR2, sFGFR3, and sFGFR4 at four concentrations of the standards and analyzed in triplicates. The percentage recovery was calculated by subtracting the baseline value from the spiked concentration and dividing the result with the spiked concentration and multiplying by 100. It was observed that the average recovery for sFGFR2 ranged from 76 - 126% (N=3), for sFGFR3 ranged from 89 - 143% (N=3), and for sFGFR4 ranged from 81 - 145% (N=3) (FIG. 2, FIG. 3, FIG. 4 and FIG. 12).

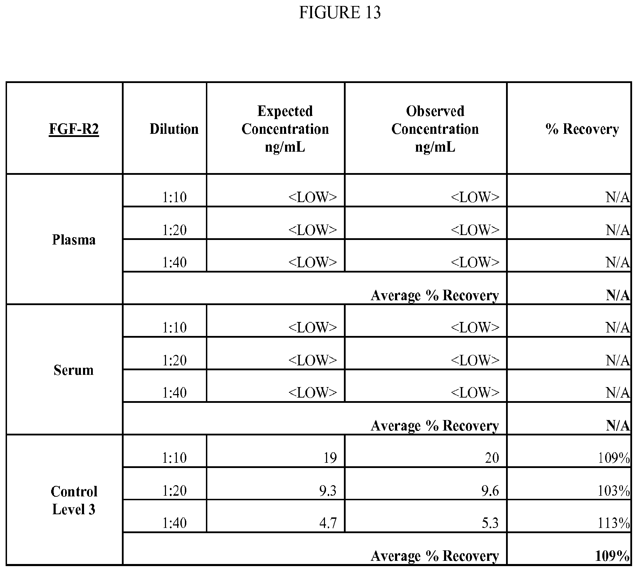

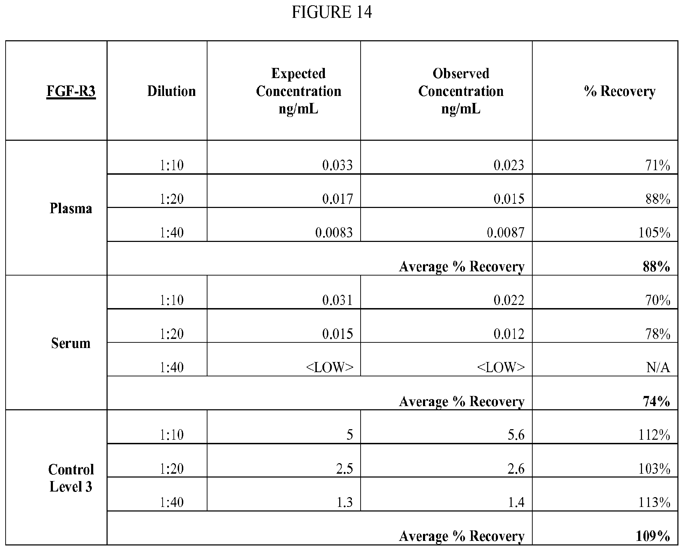

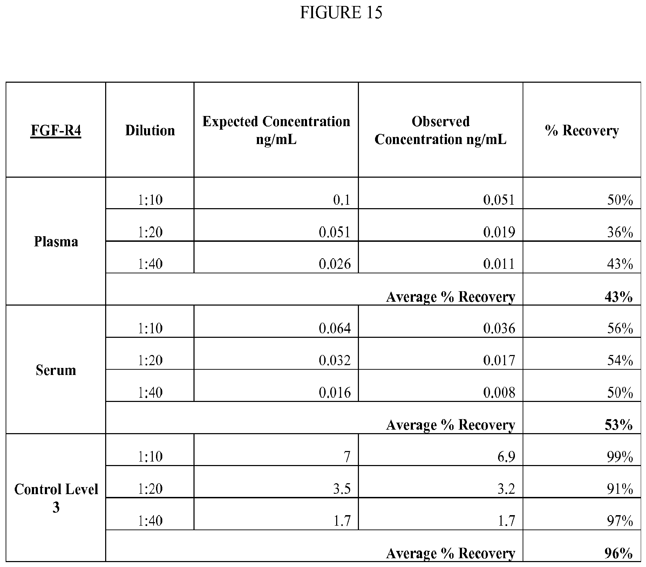

**Dilutional Linearity:** Occasionally, there can be samples that read higher than the upper range of the assay for each of the analytes. In order to get a correct value, it is important to ascertain that the samples dilute linearly in assay buffer and have a percent recovery within 70-130%. Samples were diluted two-fold in assay buffer, starting from original sample at 1:5, and then diluting to a final concentration of 1:10, 1:20, and 1:40. Dilution linearity testing was performed on neat normal serum and plasma samples, but the calculated sFGFR concentrations for these samples were below the LLOQ of the assay. Pooled normal human serum was initially spiked with recombinant sFGFR and then diluted to a final concentration of 1:10, 1:20 and 1:40. Spiked serum samples can be successfully diluted to 1:40 with an average percent recovery of 109% for sFGFR2, 109% for sFGFR3, and 96% for sFGFR4 (FIG. 13, FIG. 14 and FIG. 15).).

**Matrix Interference:** Matrix interference measures whether common components found in samples such as hemoglobin, bilirubin or triglycerides introduce error in the multiplex assay. Matrix interference was evaluated by spiking hemoglobin (up to 500 mg/dL), bilirubin (up to 20 mg/dL), and triglycerides (up to 500 mg/dL) into a control sample and determining percent recovery as observed in the spiked sample versus unspiked sample. Percentage recovery after spiking hemoglobin (up to 500 mg/dL), bilirubin (up to 20 mg/dL), and triglycerides (up to 500 mg/dL) for sFGFR2 was 89%, 91%, and 117%, for sFGFR3 was 88%, 90%, and 107%, and for sFGFR4 was 85%, 89%, and 117%, respectively.

**Stability:** During analysis, samples can go through several freeze-thaw cycles; therefore, it is important to assess the stability of the analyte after several rounds of freeze-thaw, and also determine the stability at 4 °C. Freeze-thaw stability was determined by assaying serum samples for up to three cycles of freeze-thaw and the percent recovery of each sample was determined. In addition, serum samples were also stability tested by room temperature and 4 °C incubations for 24 hours each. The incubated samples were tested along with samples that were not incubated and percent recovery was determined.

Briefly, serum (N = 3) was spiked with the recombinant standard and baseline concentration was measured for each. Samples were made to go through three cycles of freeze-thaw and percent recovery was calculated by comparing to the baseline sample. The average percent recovery for serum (N = 3) over three freeze-thaw cycles was 90.11% for sFGFR2, 84.11% for FGFR3, and 89.00% for FGFR4. Samples(N = 3) were also assessed for their antigen stability at 4 °C for 2, 4 and 24 hr time points and at room temperature for 4 and 24 hr time points. The average percent recovery for serum (N = 3) within 24 hr at room temperature and 4 °C was 83.93%, 83.33% , and 89.87% for sFGFR2, sFGFR3, and sFGFR4.

### EXAMPLE 4: Assay application

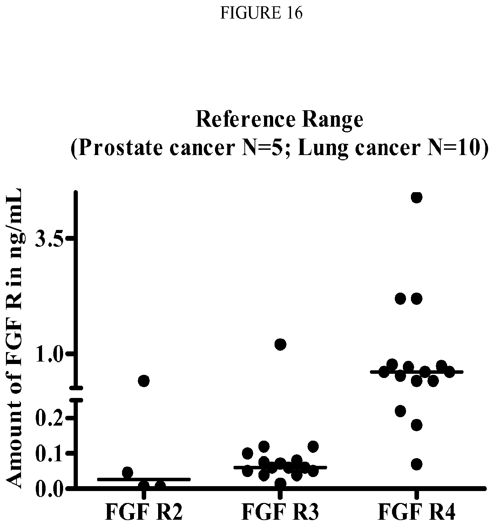

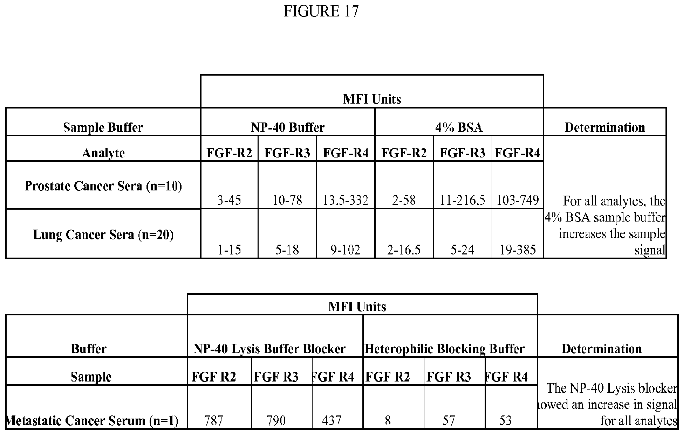

The ability of the assay to detect sFGFRs in cancer samples was assessed. FIG. 16 shows that the best sFGFR signals in the assay were obtained when the sample buffer for analyses of cancer sera contained 4% BSA and the blocking buffer contained the detergent, NP-40. After selection of the best buffers, fifteen different cancer samples were examined: five prostate cancer samples and ten lung cancer samples (FIG. 17). From those fifteen samples, sFGFR2 could only be detected in four samples with measurable amounts ranging between 0.007-0.41 ng/mL. Interestingly, sFGFR3 could be measured on all fifteen samples with measurements ranging from 0.015-1.2 ng/mL. Moreover, sFGFR4 was present in all samples at the highest concentrations ranging from 0.07-4.4 ng/mL.

## Claims

1. A kit to detect soluble fibroblast growth factor receptors (sFGFRs) in a biological sample, comprising:
a capture antibody composition specifically binding sFGFRs conjugated to the microsphere; and
a detection antibody composition specifically binding sFGFRs conjugated to a detection label;
wherein 0.14 - 494 ng/mL sFGFR2, 0.023 - 74 ng/mL of FGFR3, and 0.033 - 123 ng/mL of FGFR4 is detected in the biological sample;
wherein the capture antibody composition comprises a capture antibody specifically binding FGFR2, a capture antibody specifically binding FGFR3, and a capture antibody specifically binding FGFR4; and
wherein the detection antibody composition comprises a detection antibody specifically binding FGFR2, a detection antibody specifically binding FGFR3, and a detection antibody specifically binding FGFR4.

2. The kit of claim 1, wherein the biological sample is selected from the group consisting of blood, serum, urine, cerebrospinal fluid (CSF), plasma, lymph, saliva, sweat, pleural fluid, synovial fluid, tear fluid, bile, and pancreas secretion.

3. The kit of claim 1, wherein the capture antibody is selected from the group consisting of:
mouse anti-human FGFR2 monoclonal antibody;
mouse anti- human FGFR3 monoclonal antibody; and
mouse anti-human phospho-FGFR4 polyclonal antibody.

4. The kit of claim 1, wherein the detection antibody is selected from the group consisting of:
mouse anti-human FGFR2 monoclonal antibody;
mouse anti-human FGFR3 monoclonal antibody; and
mouse anti-human FGFR4 polyclonal antibody.

5. The kit of claim 1, wherein the microsphere is conjugated to two fluorophores.

6. The kit of claim 5, wherein the two fluorophores conjugated to the microspheres are red and infrared fluorophores.

7. The kit of claim 6, wherein the microsphere has a diameter of about 5 µm, the kit optionally further comprising a blocking buffer and an incubation buffer.

8. The kit of claim 1 to detect sFGFR2, sFGFR3, and sFGFR4 in a biological sample, comprising:
a mouse anti-human FGFR2 monoclonal antibody conjugated on a microsphere;
a mouse anti- human FGFR3 monoclonal antibody conjugated on a microsphere;
a mouse anti-human phospho-FGFR4 polyclonal antibody conjugated on a microsphere;
a mouse anti-human FGFR2 monoclonal antibody conjugated to a detection label;
a mouse anti-human FGFR3 monoclonal antibody conjugated to a detection label;
a mouse anti-human FGFR4 polyclonal antibody conjugated to a detection label;
wherein 0.14 - 494 ng/mL sFGFR2, 0.023 - 74 ng/mL of FGFR3, and 0.033 - 123 ng/mL of FGFR4 is detected in the biological sample.

9. The kit of claim 8, further comprising a blocking buffer and an incubation buffer.

10. A method of measuring sFGFR in a sample, comprising
a. providing the sample
b. measuring sFGFR in the sample using the kit of claim 9, optionally wherein the sample is obtained from a living subject.

11. A method for measuring the amount of sFGFR2, sFGFR3 and sFGFR4 in a biological sample, comprising:
a. contacting the biological sample with a capture antibody composition specifically binding sFGFR2, sFGFR3, and sFGFR4 conjugated on a microsphere;
b. contacting the biological sample with a detection antibody composition specifically binding sFGFR2, sFGFR3, and sFGFR4 coupled to a detection label;
c. measuring the signal produced by the detection label;
d. correlating the amount of the signal produced in c. to the amount of sFGFR2, sFGFR3 or sFGFR4 in the biological sample.

12. The method of claim 11, wherein the minimum detection sensitivity for
sFGFR2 is 0.14 ng/mL;
sFGFR3 is 0.023 ng/mL; and
sFGFR4 is 0.033 ng/mL.

13. The method of claim 12, wherein the biological sample is selected from the group consisting of blood, serum, urine, cerebrospinal fluid (CSF), plasma, lymph, saliva, sweat, pleural fluid, synovial fluid, tear fluid, bile, and pancreas secretion, optionally wherein each microsphere has a diameter of about 5 µm.

## Patentansprüche

1. Kit zum Nachweis löslicher Fibroblasten-Wachstumsfaktor-Rezeptoren (sFGFRs) in einer biologischen Probe, umfassend:
eine Fangantikörperzusammensetzung, die sFGFRs spezifisch bindet, die mit den Mikrokügelchen konjugiert sind; und
eine Nachweisantikörperzusammensetzung, die sFGFRs spezifisch bindet, die mit einer Nachweismarkierung konjugiert sind;
wobei 0,14 bis 494 ng/ml sFGFR2, 0,023 bis 74 ng/ml FGFR3 und 0,033 bis 123 ng/ml FGFR4 in der biologischen Probe nachgewiesen werden;
wobei die Fangantikörperzusammensetzung einen Fangantikörper, der FGFR2 spezifisch bindet, einen Fangantikörper, der FGFR3 spezifisch bindet, und einen Fangantikörper, der FGFR4 spezifisch bindet, umfasst;
wobei die Nachweisantikörperzusammensetzung einen Nachweisantikörper, der FGFR2 spezifisch bindet, einen Nachweisantikörper, der FGFR3 spezifisch bindet und einen Nachweisantikörper, der FGFR4 spezifisch bindet, umfasst.

2. Kit nach Anspruch 1, wobei die biologische Probe ausgewählt ist aus der Gruppe bestehend aus Blut, Serum, Urin, Cerebrospinalflüssigkeit (CSF), Plasma, Lymphe, Speichel, Schweiß, Pleuraflüssigkeit, Synovialflüssigkeit, Tränenflüssigkeit, Galle und Pankreas-Sekretion.

3. Kit nach Anspruch 1, wobei der Fangantikörper ausgewählt ist aus der Gruppe bestehend aus:
monoklonalem Maus-Anti-Human-FGFR2-Antikörper;
monoklonalem Maus-Anti-Human-FGFR3-Antikörper; und
polyklonalem Maus-Anti-Human-Phospho-FGFR4-Antikörper.

4. Kit nach Anspruch 1, wobei der Nachweisantikörper ausgewählt ist aus der Gruppe bestehend aus:
monoklonalem Maus-Anti-Human-FGFR2-Antikörper;
monoklonalem Maus-Anti-Human-FGFR3-Antikörper; und
polyklonalem Maus-Anti-Human-FGFR4-Antikörper.

5. Kit nach Anspruch 1, wobei das Mikrokügelchen mit zwei Fluorophoren konjugiert ist.

6. Kit nach Anspruch 5, wobei die zwei mit den Mikrokügelchen konjugierten Fluorophore rote und infrarote Fluorophore sind.

7. Kit nach Anspruch 6, wobei das Mikrokügelchen einen Durchmesser von etwa 5 µm aufweist, wobei der Kit zudem gegebenenfalls einen Blockierungspuffer und einen Inkubationspuffer umfasst.

8. Kit nach Anspruch 1 zum Nachweis von sFGFR2, sFGFR3 und sFGFR4 in einer biologischen Probe, umfassend:
einen monoklonalen Maus-Anti-Human-FGFR2-Antikörper, der an ein Mikrokügelchen konjugiert ist;
einen monoklonalen Maus-Anti-Human-FGFR3-Antikörper, der an ein Mikrokügelchen konjugiert ist;
einen polyklonalen Maus-Anti-Human-Phospho-FGFR4-Antikörper, der an ein Mikrokügelchen konjugiert ist;
einen monoklonalen Maus-Anti-Human-FGFR2-Antikörper, der mit einer Nachweismarkierung konjugiert ist;
einen monoklonalen Maus-Anti-Human-FGFR3-Antikörper, der mit einer Nachweismarkierung konjugiert ist;
einen polyklonalen Maus-Anti-Human-FGFR4-Antikörper, der mit einer Nachweismarkierung konjugiert ist;
wobei 0,14 bis 494 ng/ml sFGFR2, 0,023 bis 74 ng/ml FGFR3 und 0,033 bis 123 ng/ml FGFR4 in der biologischen Probe nachgewiesen werden.

9. Kit nach Anspruch 8, zudem umfassend einen Blockierungspuffer und einen Inkubationspuffer.

10. Verfahren zum Messen von sFGFR in einer Probe, umfassend
a. Bereitstellen der Probe
b. Messen von sFGFR in der Probe unter Verwendung des Kits nach Anspruch 9, wobei die Probe gegebenenfalls von einem lebenden Individuum erhalten wird.

11. Verfahren zum Messen der Menge von sFGFR2, sFGFR3 und sFGFR4 in einer biologischen Probe, umfassend:
a. Inkontaktbringen der biologischen Probe mit einer Fangantikörperzusammensetzung, die sFGFR2, sFGFR3 und sFGFR4 spezifisch bindet, die an ein Mikrokügelchen konjugiert sind;
b. Inkontaktbringen der biologischen Probe mit einer Nachweisantikörperzusammensetzung, die sFGFR2, sFGFR3 und sFGFR4 spezifisch bindet, die mit einer Nachweismarkierung gekoppelt sind;
c. Messen des von der Nachweismarkierung erzeugten Signals;
d. Korrelieren der Menge des in c. erzeugten Signals mit der Menge von sFGFR2, sFGFR3 oder sFGFR4 in der biologischen Probe.

12. Verfahren nach Anspruch 11, wobei die minimale Nachweisempfindlichkeit für
sFGFR2 0,14 ng/ml ist;
sFGFR3 0,023 ng/ml ist; und
sFGFR4 0,033 ng/ml ist.

13. Verfahren nach Anspruch 12, wobei die biologische Probe ausgewählt ist aus der Gruppe bestehend aus Blut, Serum, Urin, Cerebrospinalflüssigkeit (CSF), Plasma, Lymphe, Speichel, Schweiß, Pleuraflüssigkeit, Synovialflüssigkeit, Tränenflüssigkeit, Galle und Pankreas-Sekretion, wobei gegebenenfalls jedes Mikrokügelchen einen Durchmesser von etwa 5 µm hat.

## Revendications

1. Kit de détection de récepteurs de facteur de croissance des fibroblastes (sFGFR) dans un échantillon biologique, comprenant :
une composition d'anticorps de capture se liant spécifiquement aux sFGFR conjugué à la microsphère ; et
une composition d'anticorps de détection se liant spécifiquement aux sFGFR conjugué à un marqueur de détection ;
dans lequel 0,14 à 494 ng/ml de sFGFR2, 0,023 à 74 ng/ml de FGFR3, et 0,033 à 123 ng/ml de FGFR4 sont détectés dans l'échantillon biologique ;
dans lequel la composition d'anticorps de capture comprend un anticorps de capture se liant spécifiquement à FGFR2, un anticorps de capture se liant spécifiquement à FGFR3, et un anticorps de capture se liant spécifiquement à FGFR4 ; et
dans lequel la composition d'anticorps de détection comprend un anticorps de détection se liant spécifiquement à FGFR2, un anticorps de détection se liant spécifiquement à FGFR3, et un anticorps de détection se liant spécifiquement à FGFR4.

2. Kit selon la revendication 1, dans lequel l'échantillon biologique est choisi dans le groupe comprenant le sang, le sérum, l'urine, le liquide céphalo-rachidien (LCR), le plasma, la lymphe, la salive, la sueur, le liquide pleural, le liquide synovial, le liquide lacrymal, la bile et une sécrétion du pancréas.

3. Kit selon la revendication 1, dans lequel l'anticorps de capture est choisi dans le groupe constitué de :
un anticorps monoclonal de souris anti-FGFR2 humain ;
un anticorps monoclonal de souris anti-FGFR3 humain ; et
un anticorps polyclonal de souris anti-FGFR4 humain.

4. Kit selon la revendication 1, dans lequel l'anticorps de détection est choisi dans le groupe constitué de :
anticorps monoclonal de souris anti-FGFR2 humain ;
anticorps monoclonal de souris anti-FGFR3 humain ; et
anticorps polyclonal de souris anti-FGFR4 humain.

5. Kit selon la revendication 1, dans lequel la microsphère est conjuguée à deux fluorophores.

6. Kit selon la revendication 5, dans lequel les deux fluorophores conjugués aux microsphères sont des fluorophores rouges et infrarouges.

7. Kit selon la revendication 6, dans lequel la microsphère a un diamètre d'environ 5 µm, le kit comprenant facultativement en outre un tampon de blocage et un tampon d'incubation.

8. Kit selon la revendication 1 pour détecter sFGFR2, sFGFR3 et sFGFR4 dans un échantillon biologique, comprenant :
un anticorps monoclonal de souris anti-FGFR2 humain conjugué sur une microsphère ;
un anticorps monoclonal de souris anti-FGFR3 humain conjugué sur une microsphère ;
un anticorps polyclonal de souris anti-phospho-FGFR4 humain conjugué sur une microsphère ;
un anticorps monoclonal de souris anti-FGFR2 humain conjugué à un marqueur de détection ;
un anticorps monoclonal de souris anti-FGFR3 humain conjugué à un marqueur de détection ;
un anticorps polyclonal de souris anti-FGFR4 humain conjugué à un marqueur de détection ;
dans lequel 0,14 à 494 ng/ml de sFGFR2, 0,023 à 74 ng/ml de FGFR3, et 0,033 à 123 ng/ml de FGFR4 sont détectés dans l'échantillon biologique.

9. Kit selon la revendication 8, comprenant en outre un tampon de blocage et un tampon d'incubation.

10. Procédé de mesure de sFGFR dans un échantillon, comprenant
a. la fourniture de l'échantillon
b. la mesure de sFGFR dans l'échantillon au moyen du kit selon la revendication 9, facultativement dans lequel l'échantillon est obtenu à partir d'un sujet vivant.

11. Procédé de mesure de la quantité de sFGFR2, sFGFR3 et sFGFR4 dans un échantillon biologique, comprenant :
a. la mise en contact de l'échantillon biologique avec une composition d'anticorps de capture se liant spécifiquement à sFGFR2, sFGFR3 et sFGFR4 conjugués sur une microsphère ;
b. la mise en contact de l'échantillon biologique avec une composition d'anticorps de détection se liant spécifiquement à sFGFR2, sFGFR3 et sFGFR4 couplé à un marqueur de détection ;
c. la mesure du signal produit par le marqueur de détection ;
d. la corrélation de la quantité du signal produit dans c. à la quantité de sFGFR2, sFGFR3 ou sFGFR4 dans l'échantillon biologique.

12. Procédé selon la revendication 11, dans lequel la sensibilité de détection minimale pour
sFGFR2 est 0,14 ng/ml ;
sFGFR3 est 0,023 ng/ml ; et
sFGFR4 est 0,033 ng/ml.

13. Procédé selon la revendication 12, dans lequel l'échantillon biologique est choisi dans le groupe comprenant le sang, le sérum, l'urine, le liquide céphalo-rachidien (LCR), le plasma, la lymphe, la salive, la sueur, le liquide pleural, le liquide synovial, le liquide lacrymal, la bile et une sécrétion du pancréas, facultativement dans lequel chaque microsphère a un diamètre d'environ 5 µm.
